# EUROPEAN PATENT SPECIFICATION

(11) **EP 1 687 442 B1**
(45) Date of publication and mention of the grant of the patent: **09.04.2008**
(21) Application number: 03819025.2
(22) Date of filing: 17.11.2003
(51) Int. Cl.: C12Q 1/68

(54) **NUCLEIC ACID DETECTION**
NUKLEINSÄURENACHWEIS
DETECTION D'ACIDES NUCLEIQUES

(43) Date of publication of application: 09.08.2006
(73) Proprietor: Agilent Technologies, Inc., Santa Clara, CA 95051 (US)
(72) Inventor: LÜBKE, Gerd, 71088 Holzgerlingen (DE)
(74) Representative: Barth, Daniel Mathias
(86) International application number: PCT/EP2003/050844
(87) International publication number: WO 2005/049859

(56) References cited:
- EP-A- 1 186 673
- WO-A-01/57258
- US-A- 6 013 442
- KOLESAR J M ET AL: "Direct quantification of HIV-1 RNA by capillary electrophoresis with laser-induced fluorescence" JOURNAL OF CHROMATOGRAPHY B: BIOMEDICAL SCIENCES & APPLICATIONS, ELSEVIER SCIENCE PUBLISHERS, NL, vol. 697, 12 September 1997 (1997-09-12), pages 189-194, XP002083988 ISSN: 1570-0232
- BIANCHI N ET AL: "Capillary electrophoresis: detection of hybridization between synthetic oligonucleotides and HIV-1 genomic DNA amplified by polymerase-chain reaction" JOURNAL OF VIROLOGICAL METHODS, AMSTERDAM, NL, vol. 47, no. 3, 1994, pages 321-329, XP002084004 ISSN: 0166-0934

## Description

### BACKGROUND

The present invention relates to detection of a nucleic acid sequence in a mixture of different nucleic acids and a kit therefor.

WO 0157258 A discloses a direct detection and quantitation of RNA or DNA contained in a sample obtained by capillary electrophoresis of the RNA or DNA hybridized to a DNA or RNA probe of complementary sequence stabilized by the combination of a fluorophore terminally conjugated to the probe and a dye intercalating the RNA-DNA or DNA-DNA hybrid so formed.

EP 1186673 A, by the same applicant, discloses calibrating different types of signals scanned from a molecular array, or calibrating signals scanned from different molecular arrays, by employing calibrating probes that generate signals proportional to the total concentrations of labeled target molecules to which the molecular array probes are directed over an entire range of sample solutions, and molecular arrays incorporating sets of calibrating probes.

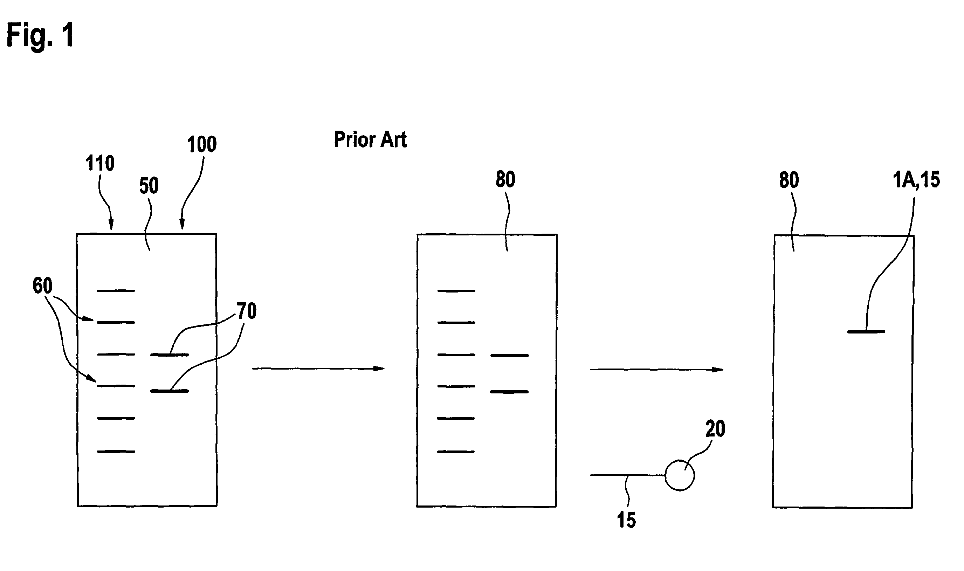

Widespread conventional techniques for the detection of nucleic acid sequences are the southern blotting technique for DNA and the northern blotting technique for RNA. At the beginning of these procedures the nucleic acid mixtures are separated in nucleic acids of different mass using gel electrophoresis, for example in an agarose or polyacrylamide gel. Following the gel electrophoresis, the different nucleic acids are preferably converted to single stranded nucleic acids. The single stranded nucleic acids are then transferred onto a microcellulose or a nylon filter and are crosslinked with the membrane using heat or UV radiation. The membrane is then blocked with a blocking reagent to saturize all unspecific binding sites of the membrane. Subsequently the nucleic acids fixed on the membrane are hybridized with a labeled nucleic acid probe, which includes a primary sequence complementary to the primary sequence of a target nucleic acid sequence. The label of the nucleic acid probe often contains ³²P-labeled phosphates, which can be detected due to their radioactivity (see for example Figure 1). The northern or southern blotting techniques therefore involve lots of different steps, e.g. gel electrophoresis, blotting onto a membrane and detection by hybridization, which are very time consuming and complicated to carry out. For the southern and northern blotting techniques different media (gels for gel electrophoresis and membranes for the blotting) are used, so that lots of different and at least partially expensive materials are used.

### DISCLOSURE

Therefore there is a need for a new method for detection of a target nucleic acid sequence in a mixture of different nucleic acids, which allows a fast and reliable detection of a target nucleic acid without the necessity to use blotting techniques. The present invention meets these needs by providing a method for detection of a target nucleic acid sequence according to the base claim 1. Favorable embodiments of the method of the invention and a kit for the detection of the target nucleic acid sequence are subjects of further claims.

Embodiments of the invention provide a fast and easy-to-handle procedure for detection of a target nucleic acid sequence, wherein the hybridizing of the target nucleic acid sequence with the probe takes place in liquid phase. The procedure in A) therefore avoids the complicated, time-consuming and also material-consuming stepproceedure of transfer of the nucleic acids onto a membrane. Furthermore an operator carrying out the embodiments of the invention usually needs less skill than an operator carrying out conventional Northern or Southern blot techniques. After the hybridizing of the target nucleic acid sequence with the labeled probe in stepA), forming a at least partially double stranded hybrid strand between the target nucleic acid and the probe, the different nucleic acids and the target nucleic acid sequence are separated in subsequent B), allowing a detection of the target nucleic acid sequence in following C). Therefore the method for detection requires the hybridizing of the probe with the target nucleic acid sequence prior to separating the nucleic acids. This sequence of the method is reversed in comparison to the conventional northern and southern blotting techniques, where the nucleic acids are separated first and then hybridized with a labeled probe.

In A1), the additional binding sites are hybridized with single-stranded nucleic acids having random primary sequences in liquid phase. The additional binding sites of the nucleic acids, which are still present after A) are often comprised of unpaired bases in single stranded areas of the nucleic acids.

The single-stranded nucleic acids can basepair with single-stranded parts of the different nucleic acids in the nucleic acid mixture and if present also with single-stranded parts of the target nucleic acid sequence, forming nucleic acid double strands. Therefore after A1) the nucleic acids in the nucleic acid mixture are mainly double-stranded, simplifying the separation of the different nucleic acids in the subsequent B). Due to A1) no retardation of the double stranded hybrid between the probe and the target nucleic acid sequence in comparison to the other nucleic acids occurs during the separation procedure in B). A retardation of the double stranded hybrid during gel electrophoresis normally takes place, when single stranded nucleic acids are still present in the nucleic acid mixture and are also subjected to gel electrophoresis, so that the important information about the size of the target sequence is lost. In practice the information about the size of the target sequence is often used as a control for the correct hybridization between the target sequence and the probe.

Advantageously short nucleic acids with a random primary sequence having a length of 6 to 14 nucleotides are provided in A1) for conversion of the single-stranded parts of the nucleic acid mixture into double-stranded parts. These short oligonucleotides are easy to synthesize and can easily be handled during A1). Due to their small size, these oligonucleotides reliably interact with single-stranded areas in the nucleic acid mixture.

In another variant , the hybridizing in A1) is carried out at roughly room temperature and the hybridizing of the probe with the target sequence in A) is carried out at a temperature between 30°C to 72°C, preferably 56°C to 72°C. A temperature between 30°C to 48°C can also be useful. A further preferred condition for hybridizing in A) is a pH range between 6 to 8.5, preferably slightly alkaline, for example pH 7.5 (e.g. TRIS EDTA buffer pH 7.5).

Due to the low temperature during hybridizing in A1) mismatches in the base pairing do not impair interaction between single-stranded areas of the different nucleic acids in the mixtures and the oligonucleotides with the random primary sequence. In contrast to the low temperature in A1) a higher temperature in A) is used in order to provide a more stringent condition for hybridizing, therefore enabling a good selectivity during the detection of the target nucleic acid sequence by the probe, reducing false signals.

A nucleic acid having a length of at least 2 times the length of the oligonucleotides with the random primary sequence can be used as a probe. When the probe is large compared to the oligonucleotides with the random sequence, it is possible to carry out A1) and A) simultaneously. Due to cooperative effects, the large probe is still able to interact with the correct target sequence and can also replace short oligonucleotides with the random primary sequence, which already have bound to the target nucleic acid sequence. This variant therefore provides the hybridization of the target nucleic acid sequence with the probe and the conversion of the single-stranded areas of the nucleic acid into double stranded nucleic acids in one go. This procedure therefore reduces the number of method sequences, allowing a faster and easier detection of the target nucleic acid sequence.

Advantageously, in A1) nucleic acids labeled with a second label are used for hybridizing, the second label being different from the first label.

Due to the different labels for the probe and for the nucleic acids having random primary sequences, the amount and the size of the target nucleic acid sequence and the amount of the total nucleic acids in the mixture can be determined using different detection methods.

It is also possible that the nucleic acids with the random primary sequence used for hybridizing in A1) are labeled with a second label after A1), the second again being different from the first label. Such a subsequent labeling of the nucleic acids can, for example, be carried out using dyes like ethidiumbromide, acridine orange, proflavin or Sybr Green®. These intercalating agents are normally used to stain double- or single-stranded nucleic acids.

It is also possible to label the oligonucleotides with the random primary sequence used in A1) by a random-oligonucleotide labeling method, developed by Feinberg and Vogelstein (Feinberg, A.P., Vogelstein, B., Anal Biochem 137, 266 - 267, 1984). Using this method random decanucleotide primers can be used for synthesis of complementary strands of template nucleic acids. The complementary strands are synthesized from the 3'-end of the random decanucleotide primers using, for example Klenow fragment of DNA polymerase I. In the presence of nucleotides, which are marked with a label, for example biotine or ³²P, labeled oligonucleotides for A1) are synthesized.

Favorably, in A2) prior to A) the mixture of different nucleic acids is denatured.

Denaturing advantageously converts the nucleic acids, which might be double-stranded into single-stranded nucleic acids, so that the hybridization in subsequent A) can occur without major difficulties. Denaturing might be carried out, for example, by heating the nucleic acid mixture to high temperatures, for example 90°C to 99°C, preferably 95°C to 99°C for a certain time, e.g. five minutes and immediately reducing the temperature afterwards e.g. by chilling on ice.

Preferably in A) a nucleic acid is used as a probe, having a stretch of 18 to 25 nucleotides being able to hybridize with the target nucleic acid sequence, this stretch having at least 80% sequence homology to the complementary sequence of the target nucleic acid sequence. Alternatively the nucleic acid probe can have at least 12 continuos nucleotides, complementary to the target nucleic acid sequence in order to ensure a good and reliable hybridization between the target nucleic acid sequence and the probe. Such nucleic acid probes can selectively detect the target nucleic acid even within a mixture of other different nucleic acids.

In another embodiment the nucleic acids are separated according to their mass in B) by using a gel electrophoresis. The gel electrophoresis can, for example, be carried out in a polyacrylamide gel or an agarose gel. This separation technique is especially suited to separate the nucleic acids in a reliable manner and in a short time.

Preferably in B) a microfluidic chip having capillaries suitable for nucleic acids electrophoresis is used for separation. The microfluidic chip can comprise, for example, a glass or silicon chip in which capillaries are etched. The capillaries can be filled with a electrophoresis medium, for example polyacrylamide or agarose and the nucleic acid mixture can be driven through the capillaries using electrophoretic and electro-osmotic forces. Using these microfluidic chips, small volumes can be analyzed very quickly. Therefore microfluidic chips are well-suited to save working time and also reduce the expenses for material.

Preferably the first and the second label are being selected from the following group:
- radioactive labels, fluorescent markers, chemoluminescence, bioluminescence, magnetic labels and antigen labels.

These kind of labels are especially suited to label nucleic acids and can easily be monitored using standard detection methods like autoradiography, fluorescence assays or antibodies.

Preferably fluorescent markers are used as the first and if present second label, wherein the fluorescent markers of the first and second label emit radiation of different wavelengths. Using this variant the detection of both the target nucleic acid sequence and the other different nucleic acids in the mixture can easily by carried out.

When fluorescent markers are used as the first and second label, both fluorescent markers emitting radiation of different wavelengths, the amount and the size of the target nucleic acid and the amount of the other different nucleic acids in the mixture can be determined via the first and second label using a spectrometer for the detection of both labels in C). This embodiment allows a simultaneous detection of both the target nucleic acid sequence and the other nucleic acids in the mixture by simply using a spectrometer e.g. a bioanalyzer instrument.

A person of ordinary skill in the art can synthesize different kinds of nucleic acid probes, depending on the target nucleic acid sequence, which has to be detected. If for example the HI-virus has to be detected in a human tissue sample, a nucleic acid probe can be designed by a person of ordinary skill in the art, which shows a high degree of complementarity in a well-conserved stretch of the HIV genome. This nucleic acid probe might still allow some mismatches in the base pairing, for example in regions of high variability within different HIV subtypes in order to allow a detection of HIV independent from its subtypes. Furthermore, additional nucleic acid probes for HIV detection can be designed by a person of ordinary skill in the art, having a high degree of complementarity even in regions of high variability in the HIV genome, therefore allowing to distinguish different subtypes of HIV.

In the following embodiments of the invention will be explained in more detail. All figures are just simplified schematic representations presented for illustration purposes only.

### BRIEF DESCRIPTION OF THE DRAWINGS

Figure 1 shows the course of separation and detection of a target nucleic acid sequence during a standard northern or southern blotting technique.

The Figures 2 and 3 depict a schematic course of subsequent method proceedure during different embodiments of the invention.

### DETAILED DESCRIPTION OF EMBODIMENTS OF THE INVENTION

Referring to Figure 1 the course of subsequent method steps of a conventional southern or northern blot is shown from left to right. At the beginning of a standard detection method for nucleic acids, the nucleic acids are separated in the line 100 of the gel 50 by gel electrophoresis (shown on the left side of the page). A DNA ladder 60 might simultaneously be applied on the gel in line 110 in order to simplify the determination of the size of the nucleic acid in the mixture. Normally only highly abundant nucleic acids are visible after staining, e. g. with ethidiumbromide, like the two bands 70, representing ribosomal RNA. After the separation of the nucleic acids the nucleic acids are transferred onto a nitrocellulose or a nylon filter 80 and are cross-linked with the membrane, as shown in the middle of Fig. 1. The transfer normally also involves the treatment of the gel with NaOH in order to convert the double stranded nucleic acids into single-stranded nucleic acids, which able to hybridize with a nucleic acid probe. The transfer of the nucleic acids from the gel onto the membrane is normally very time-consuming and also requires lots of material, for example buffer solutions. After transfer, the membrane with the single-stranded nucleic acids is frequently blocked with a blocking reagent in order to saturize all unspecific binding sites on the membrane. This blocking normally takes place by incubating the membrane with commercially available blocking reagents, e. g. Denharts solution, non-fat milk or salmon sperm DNA. Afterwards the membrane is brought into contact with a solution containing a nucleic acid probe 15 with a label 20, as shown on the left side. Normally a ³²P-label is used for northern or southern blotting techniques. The labeled nucleic acid probe can hybridize with the target nucleic acid sequence, the membrane is washed and the signal is detected, e. g. by autoradiography.

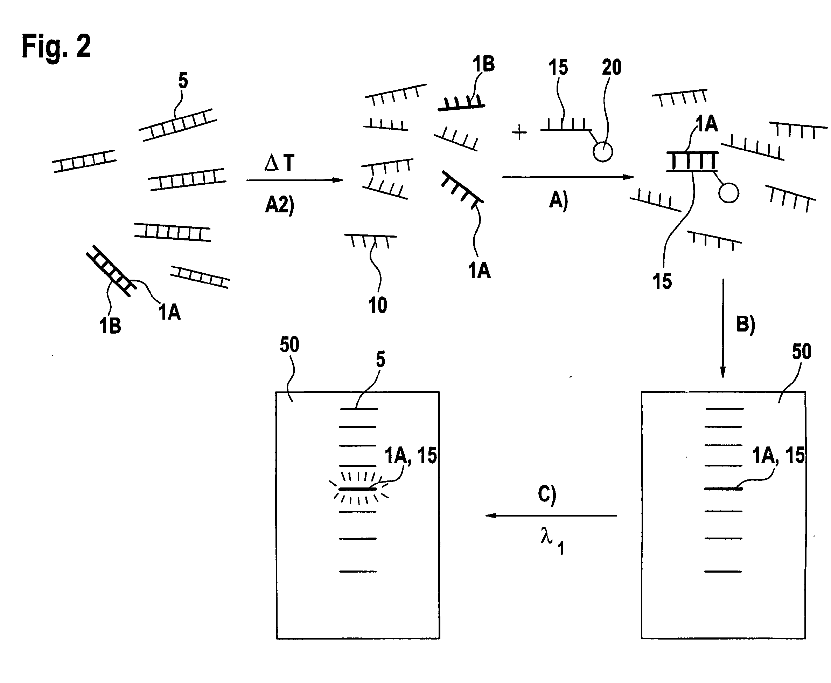

Turning now to Figure 2, one embodiment is shown. The subsequent procesing of the method is depicted in Figure 2 from left to right. At the beginning a nucleic acid mixture is used, containing different double-stranded nucleic acids 5 and also a double-stranded nucleic acid containing the target nucleic acid sequence 1A and its complementary sequence 1 B, both shown in boldfaced representation. In A2) the double-stranded nucleic acids in the nucleic acid mixture are converted to single-stranded nucleic acids, for example by heating the nucleic acid mixture to a high temperature for a short time (for example 95°C for five minutes). Afterwards the nucleic acid mixture contains mainly single-stranded nucleic acids and also the single-stranded target nucleic acid sequence 1A. Subsequently a single-stranded nucleic acid probe 15 with a first label 20 is added in A) and the probe 15 can hybridize with the single-stranded target nucleic acid 1A to form a hybrid between the probe 15 and the target nucleic acid sequence 1A. The major advantage of this embodiment in comparison to conventional methods is that A2) and A) are carried out in liquid phase and are therefore much easier to perform than the standard blotting transfer techniques shown in Figure 1. Subsequently the nucleic acid mixture can be separated, for example in a gel 50 in B) by gel electrophoresis. During C) the hybrid 1A, 15 between the target nucleic acid sequence 1A and the probe 15 can be detected, for example by using a spectrometer with the wavelength λ₁ if a fluorescent marker is used as a label. In this case the nucleic acid band containing the hybrid 1A, 15 lights up and can therefore be detected.

The denaturing of the nucleic acid mixture in A2) is compulsory, when a double stranded DNA target nucleic acid sequence has to be detected within a mixture of other double-stranded DNA molecules. It can readily be seen in Fig. 2, that the separation of the nucleic acid mixture in B) and the detection of the target nucleic acid sequence in C) are both carried out in the gel 50, so that no transfer onto a membrane is necessary.

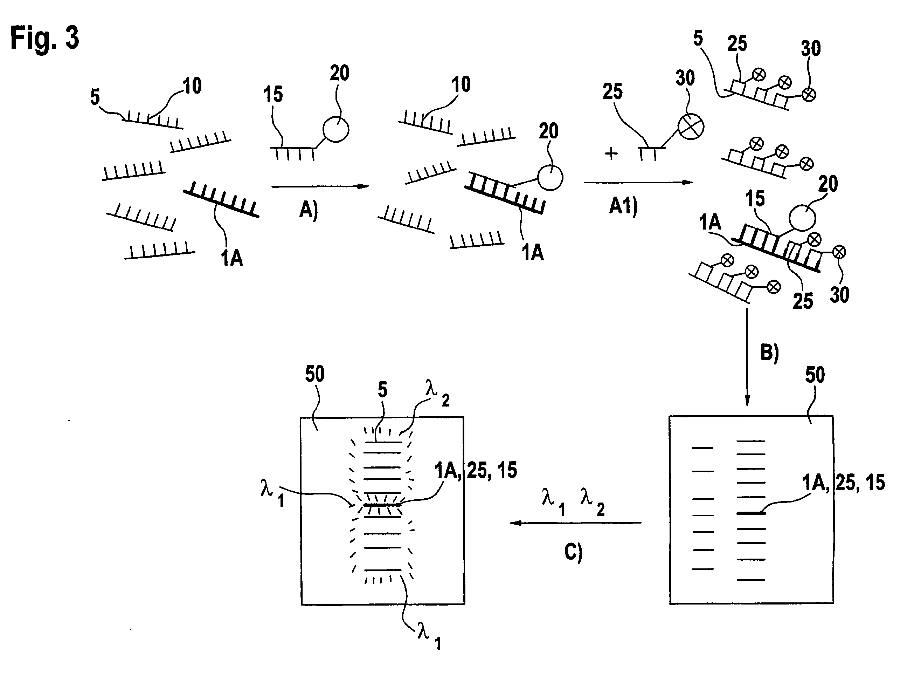

Referring now to Figure 3 another embodiment is shown from left to right. In this case the nucleic acid mixture contains single-stranded nucleic acids 5 having additional binding sites 10 and also a single-stranded target nucleic acid sequence 1A, shown again in boldfaced representation. In other cases it might be preferred to denature even single-stranded nucleic acid mixtures in order to ensure a good base pairing between the probe and the target nucleic acid sequence. In A) of this embodiment the single-stranded target nucleic acid sequence 1A is hybridized with the nucleic acid probe 15, which is labeled with a first label 20. In subsequent A1) oligonucleotides 25 with a random primary sequence, having a second label 30 are incubated with the single-stranded nucleic acids 5 in the mixture in order to bind to the additional binding sites 10, thereby converting nearly all single-stranded nucleic acids 5 into double-stranded nucleic acids. During A1) multiple oligonucleotides 25 can bind to one single-stranded nucleic acid 5 converting this nucleic acid into a double-stranded form. Additionally the oligonucleotides 25 might also bind to single-stranded regions of the hybrid between the probe 15 and the single-stranded nucleic acid target sequence 1A. A1) converts almost all of the single-stranded nucleic acids into double-stranded nucleic acids, allowing a precise mass-dependent separation of the double-stranded nucleic acids in subsequent B). The separation again might be carried out in a gel 50. Due to the conversion of A1) no shift of the signal band comprising the hybrid 1A, 25, 15 occurs during the separation of the nucleic acids in B), allowing the determination of the size of the target nucleic acid sequence. If different fluorescent markers are used as the first label 20 and the second label 30 the hybrid between the target nucleic acid sequence 1A, the probe 15 and the oligonucleotides 25 might be simultaneously detected with the other nucleic acids 5 by using a spectrometer with different wavelengths λ₁ and λ₂ in C). This special embodiment allows the determination of the amount and the size of the target nucleic acid sequence as well as the determination of the amount of the other nucleic acids 5 in the nucleic acid mixture.

### EMBODIMENTS

In order to test the feasibility of embodiments of the invention a detection was carried out, detecting the gene for the human glycerin aldehyde phosphate dehydrogenase (GADPH) in human female blood total DNA.

At the beginning the human female blood total DNA was digested using the restriction enzyme Dra I. Afterwards the DNA was concentrated by using a sodium acetate precipitation. 15 µl of sodium acetate 5 M and 175 µl ethanol were added and mixed. Afterwards the DNA was precipitated by incubating the mixture for one hour on ice. The DNA was pelleted by centrifuging 50 minutes at full speed and the DNA pellet was washed in 70% ethanol, dried and resuspended in 5 µl TE buffer (10 mM TRIS, 0.01 mM EDTA). Subsequently the digested DNA was denatured in A2) in order to convert the DNA molecules into single stranded nucleic acids by heating at 99°C for 5 minutes. Then the mixture was chilled on ice. The probe for the GADPH gene, which was labeled with the fluorescent dye BODIPY® 650/665 (available from molecular probes) and decamers with random primary sequence (available from Ambion) were both denatured in separate tubes by heating at 99°C for 5 minutes and chilling on ice. Subsequently A) was carried out by mixing the human female blood total DNA and the labeled probe, incubating for 5 minutes at 99°C, cooling down to 65°C and incubating for five minutes at 65°C. Afterwards the mixture was chilled on ice. Subsequently the decamers with the random primary sequence were added and incubated with the DNA and the labeled probe for five minutes and put on ice again in A1). The labeled GADPH probe consisted of a mixture of different probes having a medium size of 200 to 500 nucleotides, mostly being complementary to the GADPH gene and spanning the whole gene. The probes were synthesized by the random priming reaction of Feinberg and Vogelstein by using hexanucleotides as random primers. After A1) the separation of the nucleic acids in B) was carried out by transferring the nucleic acid mixture onto a DNA 12000 microfluidic chip (Agilent Technologies, Waldbronn, Germany) with 20 µM SYTO 16® (Molecular probes, Eugene, OR, USA) as a nucleic acid specific dye in the gel matrix of the chip as a second label. Using a spectrometer signals for the hybrid between the probe and the target nucleic acid sequence as well as signals for the other nucleic acids could be determined.

The scope of the invention is not limited to the embodiments shown in the figures. Indeed, variations especially concerning the combination of the different optional method features and variations concerning the design of the nucleic acid probe are possible.

## Claims

1. A method for detection of a target nucleic acid sequence (1A) in a mixture of different nucleic acids (5) having additional binding sites (10), the method comprising:
A) hybridizing the target nucleic acid sequence with a probe (15) in liquid phase, the probe having a first label (20),
A1) hybridizing the additional binding sites (10) with single stranded nucleic acids (25) having random primary sequences in liquid phase,
B) separating the different nucleic acids (1A, 5),
C) detecting the target nucleic acid (1A) by using the labeled probe (15).

2. Method according to claim 1,
- wherein short nucleic acids having a length of 6 to 12 nucleotides are provided in A1) for hybridizing.

3. Method according to claim 1 or any one of the above claims,
- wherein hybridizing in A1) is carried out at roughly room temperature, and
- hybridizing in A) is carried out at a temperature between 56°C to 72°C.

4. Method according to claim 1 or any one of the above claims,
- wherein a nucleic acid with a length of at least 10-times the length of the single stranded nucleic acids (25) with random primary sequence is used as a probe (15),
- wherein A1) and A) are carried out simultaneously.

5. Method according to claim 2 or any one of the above claims,
- wherein in A1) nucleic acids (25) labeled with a second label (30) are used for hybridizing,
- the second label (30) being different from the first label (20).

6. Method according to claim 2 or any one of the above claims,
- wherein the nucleic acids (25) used for hybridizing in A1) are subsequently labeled with a second label (30) after A1),
- the second label being different from the first label.

7. Method according to claim 1 or any of the above claims, comprising at least one of:
- prior to A) the mixture of different nucleic acids is denatured in a A2);
- in A) a nucleic acid is used as a probe (15), having a stretch of 18 to 25 nucleotides being able to hybridize with the target nucleic acid sequence (1A), this stretch having at least 80% sequence homology to the complementary sequence of the target nucleic acid sequence.

8. Method according to claim 1 or any one of the above claims, comprising at least one of:
- in B) the nucleic acids are separated according to their mass by using a gel electrophorese;
- in B) a microfluidic chip having capillaries suitable for nucleic acid electrophorese is used for separation.

9. Method according to claim 1 or any one of the above claims,
- wherein a first and if present a second label is used, each being selected from the following group:
- radioactive labels, fluorescent markers, chemoluminescence, bioluminescence, magnetic labels and antigen labels.

10. Method according to the preceeding claim,
- wherein fluorescent markers are used as the first and if present second label,
- the fluorescent markers of the first and second label emitting radiation of different wavelengths.

11. Method according to the preceeding claim,
- wherein in C) the amount and the size of the hybrid strand of the target nucleic acid (1A) and the probe (15) is determined via the first label (20) and in case the second label (30) is present, the amount of the other different nucleic acids (5) in the mixture is determined via the second label (30),
- using a spectrometer for the detection of both labels.

## Patentansprüche

1. Verfahren zum Nachweisen einer Ziel-Nukleinsäuresequenz (1A) in einer Mischung verschiedener Nukleinsäuren (5) mit weiteren Bindungsplätzen (10), wobei das Verfahren folgende Schritte aufweist:
A) Hybridisieren der Ziel-Nukleinsäuresequenz mit einer Sonde (15) in der Flüssigphase, wobei die Sonde eine erste Markierung (20) aufweist,
A1) Hybridisieren der weiteren Bindungsplätze (10) unter Verwendung von Einzelsträngen von Nukleinsäuren (25) in der Flüssigphase, deren Primärsequenzen nach dem Zufallsprinzip angeordnet sind,
B) Trennen der verschiedenen Nukleinsäuren (1A, 5),
C) Nachweisen der Zielnukleinsäure (1A) unter Verwendung der markierten Sonde (15).

2. Verfahren nach Anspruch 1,
- bei dem in A1) zum Hybridisieren kurze Nukleinsäuren mit einer Länge von 6 bis 12 Nukleotiden bereitgestellt werden.

3. Verfahren nach Anspruch 1 oder einem der obigen Ansprüche,
- bei dem das Hybridisieren in A1) ungefähr bei Raumtemperatur durchgeführt wird, und
- das Hybridisieren in A) bei einer Temperatur zwischen 56 °C und 72 °C durchgeführt wird.

4. Verfahren nach Anspruch 1 oder einem der obigen Ansprüche,
- bei dem als Sonde (15) eine Nukleinsäure verwendet wird, die zehnmal so lang ist wie die Einzelstränge von Nukleinsäuren (25), deren Primärsequenzen nach dem Zufallsprinzip angeordnet sind,
- wobei die Schritte A1) und A) gleichzeitig durchgeführt werden.

5. Verfahren nach Anspruch 2 oder einem der obigen Ansprüche,
- bei dem in Schritt A1) zum Hybridisieren Nukleinsäuren (25) verwendet werden, die mit einer zweiten Markierung (30) markiert sind,
- wobei die zweite Markierung (30) von der ersten Markierung (20) verschieden ist.

6. Verfahren nach Anspruch 2 oder einem der obigen Ansprüche,
- bei dem die in Schritt A1) zum Hybridisieren verwendeten Nukleinsäuren (25) nach dem Schritt A1) mit einer zweiten Markierung (30) markiert werden,
- wobei die zweite Markierung von der ersten Markierung verschieden ist.

7. Verfahren nach Anspruch 1 oder einem der obigen Ansprüche, wobei das Verfahren mindestens einen der folgenden Schritte aufweist:
- Denaturieren der Verbindung verschiedener Nukleinsäuren in einem Schritt A2) vor Schritt A);
- Verwenden einer Nukleinsäure als Sonde (15) in Schritt A), die eine Folge von 18 bis 25 Nukleotiden aufweist, die mit der Ziel-Nukleinsäuresequenz (1A) eine Hybridisierung eingehen können, wobei die Sequenz dieser Folge zu mindestens 80 % zu der komplementären Folge der Ziel-Nukleinsäuresequenz passt.

8. Verfahren nach Anspruch 1 oder einem der obigen Ansprüche, wobei das Verfahren mindestens einen der folgenden Schritte aufweist:
- Trennen der Nukleinsäuren in Schritt B) nach ihrer Masse unter Verwendung einer Gelelektrophorese;
- Verwenden eines Mikrofluid-Chips in Schritt B) zur Trennung, der für die Elektrophorese von Nukleinsäuren geeignete Kapillaren aufweist.

9. Verfahren nach Anspruch 1 oder einem der obigen Ansprüche,
- bei dem eine erste und gegebenenfalls eine zweite Markierung verwendet wird, die jeweils aus der folgenden Gruppe ausgewählt werden:
- radioaktive Markierungen, Fluoreszenz-, Chemolumineszenz-, Biolumineszenzmarkierungen, magnetische und Antigenmarkierungen.

10. Verfahren nach dem vorhergehenden Anspruch,
- bei dem die Fluoreszenzmarkierungen als erste und gegebenenfalls als zweite Markierung verwendet werden,
- wobei die Fluoreszenzmarkierungen der ersten und der zweiten Markierung eine Strahlung bei verschiedenen Wellenlängen emittieren.

11. Verfahren nach dem vorhergehenden Anspruch,
- bei dem in Schritt C) die Menge und die Größe des hybridisierten Strangs der Zielnukleinsäure (1A) und der Sonde (15) anhand der ersten Markierung (20) und, wenn die zweite Markierung vorhanden ist, die Menge der anderen verschiedenen Nukleinsäuren (5) in der Mischung anhand der zweiten Markierung (30) ermittelt werden,
- wobei zum Nachweis der beiden Markierungen ein Spektrometer verwendet wird.

## Revendications

1. Procédé de détection d'une séquence d'acides nucléiques cibles (1A) dans un mélange de différents acides nucléiques (5) ayant des sites de liaison (10) supplémentaires, le procédé comprenant les étapes suivantes :
A) l'hybridation de la séquence d'acides nucléiques cibles avec une sonde nucléique (15) en phase liquide, la sonde nucléique ayant une première marque (20) ;
A1) l'hybridation des sites de liaison (10) supplémentaires avec des acides nucléiques simple brin (25) ayant des séquences primaires aléatoires en phase liquide ;
B) la séparation des différents acides nucléiques (1A, 5) ;
C) la détection de l'acide nucléique cible (1A) en utilisant la sonde nucléique marquée (15).

2. Procédé suivant la revendication 1,
- dans lequel des acides nucléiques courts ayant une longueur de 6 à 12 nucléotides sont fournis à l'étape A1) pour l'hybridation.

3. Procédé suivant la revendication 1 ou suivant l'une quelconque des revendications précédentes,
- dans lequel l'hybridation à l'étape A1) est exécutée plus ou moins à température ambiante, et
- l'hybridation à l'étape A) est exécutée à une température comprise entre 56 et 72 °C.

4. Procédé suivant la revendication 1 ou suivant l'une quelconque des revendications précédentes,
- dans lequel un acide nucléique ayant une longueur d'au moins 10 fois la longueur des acides nucléiques simple brin (25) avec une séquence primaire aléatoire est utilisé comme sonde nucléique (15) ;
- dans lequel les étapes A1) et A) sont exécutées simultanément.

5. Procédé suivant la revendication 2 ou suivant l'une quelconque des revendications précédentes,
- dans lequel à l'étape A1) des acides nucléiques (25) marqués d'une deuxième marque (30) sont utilisés pour l'hybridation ;
- la deuxième marque (30) étant différente de la première marque (20).

6. Procédé suivant la revendication 2 ou suivant l'une quelconque des revendications précédentes,
- dans lequel les acides nucléiques (25) utilisés pour l'hybridation à l'étape A1) sont ensuite marqués d'une deuxième marque (30) après l'étape A1) ;
- la deuxième marque étant différente de la première marque.

7. Procédé suivant la revendication 1 ou suivant l'une quelconque des revendications précédentes, comprenant au moins une des étapes suivantes :
- avant l'étape A) le mélange de différents acides nucléiques est dénaturé à une étape A2) ;
- à l'étape A) un acide nucléique est utilisé comme sonde nucléique (15), ayant un tronçon de 18 à 25 nucléotides étant à même de s'hybrider avec la séquence d'acides nucléiques cibles (1A), ce tronçon présentant au moins une homologie séquentielle de 80 % avec la séquence complémentaire de la séquence d'acides nucléiques cibles.

8. Procédé suivant la revendication 1 ou suivant l'une quelconque des revendications précédentes, comprenant au moins une des étapes suivantes :
- à l'étape B) les acides nucléiques sont séparés selon leur masse en utilisant une électrophorèse sur gel ;
- à l'étape B) une puce microfluidique comportant des capillaires se prêtant à l'électrophorèse des acides nucléiques est utilisée pour la séparation.

9. Procédé suivant la revendication 1 ou suivant l'une quelconque des revendications précédentes,
- dans lequel une première marque et, si elle est présente, une deuxième marque sont utilisées, chacune étant sélectionnée parmi le groupe suivant :
- des traceurs, des marqueurs fluorescents, la chimioluminescence, la bioluminescence, des marques magnétiques et des antigènes.

10. Procédé suivant la revendication précédente,
- dans lequel des marqueurs fluorescents sont utilisés comme première marque et, si elle est présente, comme deuxième marque ;
- les marqueurs fluorescents de la première marque et de la deuxième marque émettant un rayonnement de longueurs d'onde différentes.

11. Procédé suivant la revendication précédente,
- dans lequel à l'étape C) la quantité et la taille du brin hybride de l'acide nucléique cible (1A) et de la sonde nucléique (15) sont déterminées par la première marque (20) et, dans le cas où la deuxième marque (30) est présente, la quantité des autres différents acides nucléiques (5) dans le mélange est déterminée par la deuxième marque (30) ;
- en utilisant un spectromètre pour la détection des deux marques.
